(19) Europäisches Patentamt
European Patent Office
Office européen des brevets

(11) Numéro de publication : **0 074 909**
**B1**

(12) **FASCICULE DE BREVET EUROPÉEN**

(45) Date de publication du fascicule du brevet : **26.06.85**

(21) Numéro de dépôt : **82401683.6**

(22) Date de dépôt : **16.09.82**

(51) Int. Cl.[4] : **A 61 K 31/70**, A 61 K 31/52, A 61 K 31/44, A 61 K 31/505, A 61 K 31/545, C 07 H 19/16, A 61 K 45/06 // (A61K31/70, 31:52, 31:505, 31:455, 31:44, 31:405)

(54) **Nouvelles compositions à base de 5-hydroxytryptophane, leur procédé de préparation et médicaments les contenant.**

(30) Priorité : **16.09.81 FR 8117492**
**11.08.82 FR 8213980**

(43) Date de publication de la demande :
**23.03.83 Bulletin 83/12**

(45) Mention de la délivrance du brevet :
**26.06.85 Bulletin 85/26**

(84) Etats contractants désignés :
**AT BE CH DE FR GB IT LI LU NL SE**

(56) Documents cités :
**DE-A- 2 841 170**
**FR-A- 2 023 937**
**FR-A- 2 081 539**
**FR-A- 2 499 076**
**GB-A- 1 180 635**
**GB-A- 1 535 778**
**MARTIN NEGWER: "Organisch-chemische Arzneimittel und ihre Synonyma" 5e édition, vol. II, 1978, Akademie-Verlag, Berlin;**
**Le dossier contient des informations techniques présentées postérieurement au dépôt de la demande et ne figurant pas dans le présent fascicule.**

(73) Titulaire : **S.A. PANMEDICA Société dite:**
**Zone Industrielle - Ilot J**
**F-06510 Carros (FR)**

(72) Inventeur : **Laruelle, Claude**
**Avenue Bellevue**
**F-06270 Villeneuve Loubet (FR)**
Inventeur : **Lepant, Marcel**
**l'Escoundu Allée Clairefontaine**
**F-06140 Vence (FR)**

(74) Mandataire : **Ores, Irène et al**
**CABINET ORES 6, Avenue de Messine**
**F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 074 909 B1

Jouve, 18, rue St-Denis, 75001 Paris, France

**Description**

La présente invention est relative à de nouvelles compositions pharmaceutiques contenant le 5-hydroxy-tryptophane.

On connaît l'importance du rôle de la sérotonine comme médiateur chimique intercellulaire cérébral (cf. notamment le Brevet Français de la Demanderesse n° 2 499 076). On a pu mettre en évidence que des états déficitaires en sérotonine étaient à l'origine de certains dérèglements du sommeil, de syndromes dépressifs, de phénylcétonurie. L'apport de 5-hydroxytryptophane (5-HTP) précurseur direct de la sérotonine permet de compenser ce déficit (on considère que la sérotonine est synthétisée à partir du tryptophane alimentaire selon la chaine métabolique suivante : tryptophane → 5-hydroxytryptophane → 5-hydroxytryptamine). L'apport en 5-HTP est en effet préférable à l'administration d'inhibiteur de monoamine oxydase qui bloque aussi bien la dégradation de la sérotonine que celle des autres amines biogènes. L'administration du 5-HTP comme précurseur immédiat de la sérotonine présente toutefois un inconvénient important : le rendement global en sérotonine cérébrale effectivement synthétisée par rapport à la quantité de 5-hydroxytryptophane administrée est très faible. Ce taux de transformation est très faible pour diverses raisons ; entre autres, il est dû à un passage médiocre des barrières gastrointestinales et hématoencéphaliques, à une importante décarboxylation au niveau périphérique et à un catabolisme hépatique élevé. Cette perte peut être partiellement compensée par administration simultanée d'un inhibiteur de décarboxylase périphérique, mais cette adjonction entraîne d'autres inconvénients.

Dans le cadre d'une première phase de ses recherches, (cf. le Brevet Français 2 499 076 déjà cité), la Demanderesse a mis au point des dérivés de 5-HTP répondant à la formule générale I ci-après :

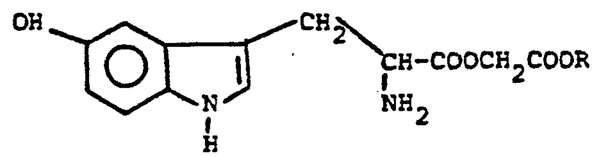

(I)

dans laquelle

R représente un atome d'hydrogène, un groupement alkyle (en $C_1$ à $C_{12}$), ou des groupements alicycliques, monocycliques ou polycycliques renfermant de 5 à 16 atomes de carbone,

lesquels dérivés ont permis d'augmenter considérablement le taux de sérotonine endogène dans le cerveau.

En poursuivant ses travaux de recherches et d'investigation dans ce domaine, la Demanderesse a pu mettre au point d'autres compositions et complexes à base de 5-HTP, lesquels non seulement permettent de réduire considérablement la déperdition de 5-HTP, mais potentialisent de façon surprenante l'effet de ce dernier.

On sait d'autre part que les nucléotides cycliques interviennent dans le mécanisme d'action des neurotransmetteurs au niveau pré- et post-synaptique [cf. par exemple GREENGARD — NATURE 260-101 (1976)], certains d'entre eux comme l'inosine et l'hypoxanthine pouvant constituer les ligands endogènes des récepteurs des benzodiazépines [P. SKOLNICK — PROC. NATL. AC. SC. USA *76* 1 515-18 (1979)].

On connaît également un certain nombre d'associations contenant le 5-HTP (ou analogues) et notamment le Brevet français 2 081 539, les Brevets britanniques 1 180 635 et 1 535 778, le Brevet allemand 2 841 170 et le Brevet français 2 023 937.

Toutefois dans ces Publications il s'agit soit d'un sel d'α céto glutarate de pyridoxine et de 5-HTP dans lequel la salification du 5-HTP est effectuée sur la fonction δ caboxylique de l'acide α céto glutarique (Brevet français 2 023 937) ; soit de tryptophane et non de 5-hydroxy-tryptophane (Brevets britanniques 1 180 635 et 1 535 778, Brevet allemand 2 841 170), or les analogies entre les propriétés physico-chimiques de ces deux composés sont inexistantes ou très faibles.

La présente invention a pour objet de nouvelles compositions pharmaceutiques capables de corriger le déficit du métabolisme de la sérotonine, caractérisées en ce qu'elles sont constituées par une association de 5-HTP avec des dérivés des bases puriques et/ou pyrimidiques et/ou pyridiques libres à l'exception de celles de ces bases qui sont substituées ou estérifiées.

La Demanderesse a en effet constaté que la combinaison du 5-HTP avec un hétérocycle purique, pyridique ou pyrimidique a permis d'augmenter considérablement le taux cérébral du 5-HTP, de la sérotonine et celui de l'acide 5-hydroxy-indolacétique (5-HIAA) qui est le métabolite principal de la sérotonine.

La présence de ces bases puriques, pyridiques ou pyrimidiques modifie le mécanisme enzymatique de dégradation du 5-HTP par la 5-hydroxytryptophane décarboxylase (présente dans le rein, le foie et l'estomac), ce qui permet de réduire considérablement les quantités du 5-HTP administré lors des traitements destinés à compenser l'abaissement du taux de la sérotonine cérébrale.

Selon un mode de réalisation avantageux de l'objet de l'invention, l'association 5-HTP-base est une association équimoléculaire. Cette association peut se présenter aussi bien sous la forme d'un complexe que d'un sel de 5-HTP avec la base purique et/ou pyrimidique et/ou pyridique à l'exception de celles de ces bases qui sont substituées ou estérifiées.

Conformément à l'invention, le 5-HTP peut être utilisé sous la forme d'un quelconque de ses énantiomères, la forme (L), la forme (D) ou le mélange racémique (DL).

Parmi les bases, les dérivés suivants sont particulièrement préférés pour leur effet potentialisateur de l'activité sérotoninergique du 5-hydroxytryptophane : l'inosine, la théophylline, la théobromine, l'allopurinol, la pyridoxine, l'hypoxanthine, l'acide folique, l'adénine, le nicotinamide, la caféine et l'acide orotique.

En interprétant les résultats cliniques de ces remarquables médicaments, la Demanderesse a pu constater que le complexe Inosine-5-HTP et notamment le complexe Inosine-(L) 5-HTP triple les taux cérébraux du 5-HT P et de l'acide 5-hydroxy-indolacétique (5-HIAA) qui est le métabolite principal de la sérotonine. La diminution de prise en 5-HTP n'est pas seulement intéressante du point de vue économique, mais elle a également un important avantage clinique, puisque l'on sait que l'administration prolongée de fortes doses de 5-HTP entraîne souvent des lésions rénales.

Cette excellente réponse pharmacologique du complexe Inosine-(L) 5-HTP et la quasi absence d'activité du (D) 5-HTP a amené la Demanderesse à étudier un nouveau procédé simple et économique de préparation du complexe Inosine-(L) 5-HTP et ceci à partir d'un produit industriel racémique à savoir le (D-L) 5-HTP.

L'on sait que les procédés de séparation des isomères optiquement actifs à partir d'un mélange racémique d'un acide aminé font intervenir des techniques et des réactifs coûteux. Ainsi, par exemple, pour isoler le (L) 5-hydroxytryptophane du mélange racémique KYOWA HAKKO KOGYO (Brevet japonais 75.58061) on fait réagir de D-thréo-1 (paranitrophényl) 2-amino-1,3 propane diol. L'isomère -L- de ce dernier réactif est également utilisé par SANKYO (Brevet japonais 73.91063) pour séparer les diastéréoisomères du (DL) 5-HTP après blocage des fonctions réactives. RINDERKNECHT [Helv. CHIM. ACTH. 47 (8) 2 403 (19)] utilise successivement la quinine et la quinidine pour séparer les deux diastéréoisomères.

Toutes les techniques de l'Art antérieur préconisent donc de bloquer les fonctions réactives du (DL) 5-HTP, de faire agir un dérivé optiquemnt actif, de séparer les couples de diastéréoisomères, puis enfin de débloquer les groupes protecteurs. L'ensemble de ces opérations est long et coûteux.

La présente invention s'est par conséquent fixé pour but de pourvoir à un procédé de préparation du complexe Inosine-(L) 5-HTP qui répond mieux aux nécessités de la pratique que les procédés antérieurement connus notamment en ce qu'il permet l'obtention de ce complexe sans la séparation préalable du dérivé (L).

La présente invention a également pour objet un procédé de préparation du complexe Inosine-(L) 5-HTP pratiquement pur, caractérisé en ce que l'on fait réagir les quantités équimoléculaires de l'inosine et du (DL) 5-HTP du commerce en solution aqueuse à une température voisine de 70 °C, en ce qu'on laisse refroidir le mélange réactionnel en 4 à 5 heures à la température ambiante puis en maintenant pendant environ 48 heures, à une température voisine de 5 °C et en ce que l'on sépare les cristaux purs du complexe Inosine-(L) 5-HTP obtenus.

Cette méthode de purification suivie de racémisation permet d'éviter de fortes colorations dues au chauffage d'inosine.

Outre les dispositions qui précèdent, l'invention comprend encore d'autres dispositions qui ressortiront de la description qui va suivre.

La présente invention pourra être mieux comprise à l'aide du complément de description qui va suivre, dans lequel on trouvera des exemples de préparation des compositions conformes à la présente invention ainsi qu'un compte-rendu d'expérimentations pharmacologiques.

Il doit être bien entendu, toutefois, que ces exemples et ce compte-rendu d'expérimentations pharmacologiques sont donnés uniquement à titre d'illustration de l'objet de l'invention, mais n'en constituent en aucune manière une limitation.

## Exemples de préparation

### Exemple 1

Inosinate de (L) 5-HTP

On prépare 134 g d'inosine et 110 g de (DL) 5-HTP que l'on ajoute à 20 litres d'eau portée à 70 °C. On laisse revenir la température du mélange réactionnel en 5 heures à la température ambiante. La cristallisation s'amorce spontanément puis elle est continuée par refroidissement à 5 °C pendant 48 heures. On filtre les cristaux à + 5 °C et on lave avec deux fois 500 ml d'eau glacée. Après séchage, on obtient le complexe équimoléculaire pur de Inosine-(L) 5-HTP (sous forme de cristaux monohydratés) avec un rendement de 83 % calculé par rapport au diastéréoisomère (L) présent dans le 5-HTP racémique de départ.

Les caractéristiques du produit obtenu sont :

$[\alpha]_D^{25}$ = 37,7 °C (C = 0,2, eau)
— point de fusion : 191 °C
— teneur en eau (Karl FISCHER) = 3,5 %.

Le dosage quantitatif des constituants par chromatographie liquide à haute performance indique :
— 5-HTP = 43,5 %
— Inosine = 53 %

L'analyse élémentaire des éléments C, H et N est conforme à la composition : Inosine-5-HTP-1$H_2O$.
La pureté optique du complexe (déterminé après isolement du 5-HTP sur résine cationique) est supérieure à 98 %.

Exemple 2

Inosinate de (DL) 5-hydroxytryptophane

On dissout 22 g (0,1 mole) de (DL) 5-hydroxytryptophane dans le minimum d'eau distillée à 50 °C, soit environ 1 litre et on ajoute 27 g (0,1 mole) d'inosine (ou 9 β D ribofuranosyl hypoxanthine). Après refroidissement, on filtre le composé, qui cristallise avec une molécule d'eau (p. f. 195 °C).
Analyse élémentaire :
calculé : C = 49,80 % H = 5,14 % N = 16,60 %
trouvé : C = 50 % H = 5,10 % N = 16,43 %

Exemple 3

Sel de théophylline du (DL) 5-hydroxytryptophane

On dissout dans 2 litres 11,0 g (0,05 mole) de (DL) 5-hydroxytryptophane et 9,0 g (0,05 mole) de théophylline (3,7-dihydro-1,3-diméthyl-1 H-purine-2,6 dione). Après quelques heures au réfrigérateur, on filtre les cristaux formés et on sèche sous vide à l'abri de la lumière (p. f. 265 °C). Solubilité aqueuse à 20 °C = 0,1 %.
Analyse élémentaire :
calculé : C = 55,38 % H = 5,13 % N = 21,5 %
trouvé : C = 55,45 % H = 5,07 % N = 21,7 %.

Exemple 4

Sel de théophylline du (L) 5-hydroxytryptophane

On dissout 0,05 mole, soit 9,0 g de théophylline et 0,05 mole soit 11 g de (L) 5-hydroxytryptophane dans environ 2 litres d'eau. Après quelques jours au réfrigérateur, on filtre les cristaux et on sèche à l'abri de la lumière (p. f. 265 °C). Solubilité aqueuse à 20 °C = 0,35 %.

Exemple 5

Complexe équimoléculaire du (L) 5-hydroxy-tryptophane et de l'allopurinol

On dissout dans le minimum d'eau bouillante 2,2 g (0,01 mole) de L 5-hydroxytryptophane et 1,4 g d'allopurinol [4 hydroxypyrazolo (3,4-d) pyrimidine] (0,01 mole). Après refroidissement et plusieurs jours au réfrigérateur, on filtre les cristaux et on sèche à l'abri de la lumière (pf. > 290 °C).
Analyse élémentaire :
calculé : C = 53,3 % H = 5,5 % N = 23,3 % O = 17,8 %
trouvé : C = 53,1 % H = 5,7 % N = 23,3 % O = 18 %

Exemple 6

Complexe équimoléculaire de la pyridoxine avec le (DL) 5-hydroxytryptophane

On dissout les quantités équimoléculaires de (DL) 5-HTP et de chlorhydrate de pyridoxine (5-hydroxy-6 méthyl 3-4 pyridine diméthanol) dans le minimum d'eau, soit environ 15 litres par mole. Après évaporation de l'eau, le résidu est repris à l'alcool. On obtient ainsi 90 % du complexe équimoléculaire bien cristallisé de p. f. = 175 °C, très soluble dans l'eau.

Exemple 7

Complexe équimoléculaire de la pyridoxine avec le (L) 5-hydroxytryptophane

On dissout dans les conditions de l'Exemple 6 ci-dessus, le (L) 5-HTP et le chlorhydrate de

pyridoxine. Après reprise à l'alcool du résidu d'évaporation, on obtient 90 % du complexe équimoléculaire bien cristallisé de p. f. 165 °C et $[\alpha]_D^{25} = -9°$ (0,2 % eau).

Analyse élémentaire :

calculé : C = 53,58 % H = 5,64 % N = 9,8 % Cl = 8,34 %

trouvé : C = 53,50 % H = 5,42 % N = 9,62 %

Exemple 8

Complexe équimoléculaire de l'hypoxanthine avec le (L) 5-hydroxytryptophane

On dissout 13,6 g (0,1 mole) d'hypoxanthine (1-7-dihydropurine-6-one) dans un litre d'eau bouillante et on ajoute 22,0 g (0,1 mole) de (L) 5-hydroxytryptophane. La solution est immédiatement concentrée sous pression réduite et le résidu sec est repris par de l'éthanol chaud puis séché. On obtient un complexe équimoléculaire d'hypoxanthine et de L-5-hydroxytryptophane.

Analyse élémentaire :

calculé : C = 53,3 % H = 5,5 % N = 23,3 % O = 17,8 %

trouvé : C = 53,5 % H = 5,5 % N = 23,3 % O = 17,6 %

Exemple 9

Association équimoléculaire de (L) 5-hydroxytryptophane et d'acide folique

On mélange soigneusement 11 g de (L) 5-hydroxytryptophane et 22 g d'acide folique (Acide N-[4-2-amino-1,4 dihydro-4 oxo-6-ptéridinylméthylaminobenzoyl]-L-glutamique), et on vérifie par les techniques appropriées l'homogénéité du mélange.

Exemple 10

Sel de nicotinamide du (L) 5-hydroxytryptophane

On met en suspension 22,0 g (0,1 mole) de (L) 5-hydroxytryptophane dans 200 ml d'eau à 50 °C et on ajoute 12,2 g (0,1 mole) de nicotinamide. Les produits passent rapidement en solution, on évapore sous vide et après séchage, on obtient le dérivé désiré.

Analyse élémentaire :

calculé : C = 59,6 % H = 5,2 % N = 16,4 % O = 18,7 %

trouvé : C = 59,5 % H = 5,3 % N = 16,4 % O = 18,5 %

Exemple 11

Sel de théobromine du (L) 5-hydroxytryptophane

On dissout 9,0 g de théobromine (0,05 mole) (3,7-dihydro-3,7-diméthyl-1H-purine-2,6 dione) et 11 g (0,05 mole) de (L) 5-hydroxytryptophane dans 500 ml d'eau chaude. On refroidit et après quelques jours à 0 °C on filtre les cristaux formés et on sèche sous vide [pf. > 250 °C (dec) — cristaux monohydratés].

Analyse élémentaire :

calculé : C = 54,0 % H = 5,0 % N = 21 % O = 20 %

trouvé : C = 54,2 % H = 5,1 % N = 21 % O = 19,8 %

Exemple 12

Sel de caféine du (L) 5-hydroxytryptophane

On dissout 10 g de caféine (0,05 mole) (1,3,7 triméthyl-2,6-dioxopurine) et 11 g (0,05 mole) de (L) 5-hydroxytryptophane dans 200 ml d'eau à 80 °C, on refroidit et après quelques jours à 0 °C on filtre les cristaux et on sèche sous vide.

Analyse élémentaire :

calculé : C = 55,1 % H = 5,3 % N = 20,3 % O = 19,3 %

trouvé : C = 55 % H = 5,2 % N = 20,3 % O = 19,5 %

Compte-rendu de l'étude pharmacologique

Cette étude a été réalisée chez le rat et a permis de déterminer l'efficacité des compositions conformes à la présente invention sur le métabolisme cérébral de la sérotonine et a permis de mettre en évidence la potentialisation de l'effet sérotoninogène par rapport au (L) 5-HTP de référence (les bases puriques, pyrimidiques et pyridiques sont, quant à elles, totalement dépourvues de cette activité).

Les animaux sont des rats mâles adultes de la souche OFA pesant 160 à 180 g. Ils sont soumis à un cycle lumineux alternatif de 12 h d'éclairage et 12 h d'obscurité.

Les différents dérivés selon l'invention ont été administrés dans l'eau distillée par voie intragastrique à des doses de 10, 25 et 50 mg/kg. Après une heure, les rats sont décapités et les teneurs de 5-hydroxytryptamine, de 5-HIAA et de 5-hydroxytryptophane dans les cerveaux sont déterminés par les méthodes publiées par G. CURZON et COLL. (Brit. J. Pharmaco. 39 653 (1970)).

Les résultats de ces déterminations ont été rassemblés dans le tableau I ci-après : ils représentent les pourcentages d'augmentation des taux endogènes cérébraux à 10, 25, 50 mg/kg du (L) 5-HTP pur (témoin) ou des associations conformes à la présente invention par rapport à un lot de rats témoins.

(Voir Tableau I, page 7)

Tableau I

| Dérivés | L 5 HTP | | | 5 HT | | | 5 HIAA | | | 5 HT + 5 HIAA | | |
|---|---|---|---|---|---|---|---|---|---|---|---|---|
| | 10 mg/kg | 25 mg/kg | 50 mg/kg | 10 mg/kg | 25 mg/kg | 50 mg/kg | 10 mg/kg | 25 mg/kg | 50 mg/kg | 10 mg/kg | 25 mg/kg | 50 mg/kg |
| (L) 5 HTP pur | 120 | 260 | 650 | 10 | 25 | 35 | 40 | 110 | 300 | 55 | 110 | 180 |
| Produit obtenu selon l'exemple 1 | 135 | 1 755 | 2 665 | 2 | 100 | 70 | 165 | 290 | 1 010 | 100 | 195 | 545 |
| Exemple 2 | 110 | 1 665 | 1 955 | 2 | 80 | 110 | 135 | 345 | 1 190 | 85 | 220 | 700 |
| Exemple 3 | 35 | 780 | 870 | — | 20 | 4 | 60 | 175 | 285 | 35 | 100 | 160 |
| Exemple 4 | 55 | 1 200 | 1 710 | — | 25 | 35 | 85 | 270 | 870 | 75 | 150 | 490 |
| Exemple 6 | 115 | 230 | 1 575 | — | 30 | 60 | 20 | 90 | 360 | 8 | 65 | 210 |
| Exemple 7 | 190 | 420 | 2 500 | 20 | 80 | 25 | 35 | 230 | 755 | 40 | 165 | 390 |
| Exemple 5 | 108 | 830 | 2 100 | 15 | 70 | 115 | 90 | 100 | 765 | 60 | 85 | 475 |
| Exemple 9 | 180 | 1 700 | 2 820 | — | 35 | 140 | 190 | 220 | 1 190 | 110 | 130 | 720 |

Il résulte de l'étude qui précède que les compositions conformes à la présente invention permettent de réaliser une économie considérable en 5-HTP et de potentialiser remarquablement les effets de ce dernier : cette amélioration est en moyenne comprise entre 200 et 300 % et peut atteindre 500 %.

Toxicité des compositions conformes à la présente invention

Les toxicités de sels et des complexes du 5-HTP conformes à la présente invention sont très faibles comme en témoignent les $DL_{50}$ per os/souris rassemblées dans le Tableau 2 ci-après :

Tableau 2

| | Produit de l'exemple 10 | Produit de l'exemple 4 | Produit de l'exemple 12 | Produit de l'exemple 9 | Produit de l'exemple 5 |
|---|---|---|---|---|---|
| $DL_{50}$/per os mg/kg | > 2 000 | 1 000 | > 2 000 | 1 000 | 1 000 |

L'intérêt des compositions conformes à la présente invention est mis immédiatement en évidence par l'augmentation considérable de l'index thérapeutique, augmentation qui présente le double avantage d'améliorer la réponse sur le plan thérapeutique et de diminuer le coût du traitement par l'économie réalisée sur le dosage du 5-THP.

Les médicaments conformes à la présente invention, dont les doses d'administration journalières peuvent varier de 1 à 100 mg par kg de poids corporel, peuvent être administrés aussi bien par voie orale (gélules, comprimés, solutions ou suspensions buvables) que par voie injectable.

**Revendications**

1. Compositions pharmaceutiques capables de corriger le déficit du métabolisme de la sérotonine, caractérisées en ce qu'elles sont constituées par une association de 5-HTP avec des bases puriques et/ou pyrimidiques et/ou pyridiques à l'exception de celles de ces bases qui sont substituées ou estérifiées.

2. Composition selon la Revendication 1, caractérisée en ce que l'association 5-HTP hétérocycle azoté est une association équimoléculaire.

3. Composition selon l'une quelconque des Revendications 1 et 2, caractérisée en ce que le 5-HTP et la base purique et/ou pyrimidique et/ou pyridique forment un complexe.

4. Composition selon l'une quelconque des Revendications 1 et 2, caractérisée en ce que le 5-HTP et la base purique et/ou pyrimidique et/ou pyridinique forment un sel.

5. Composition selon l'une quelconque des Revendications 1 à 4, caractérisée en ce que le 5-HTP peut être utilisé sous la forme de l'un quelconque de ses énantiomères, la forme (L), la forme (D) ou le mélange racémique (D L).

6. Composition selon l'une quelconque des Revendications 1 à 5, caractérisée en ce que la base formant un sel ou un complexe avec l'HTP est pris dans le groupe qui comprend l'inosine, la théophylline, la théobromine, l'allopurinol, l'hypoxanthine, l'acide folique, le nicotinamide, la caféine et l'acide orotique.

7. Médicaments constitués par ou contenant les compositions selon l'une quelconque des Revendications 1 à 6.

8. Procédé de préparation du complexe inosine-(L)-5-HTP, caractérisé en ce que l'on fait réagir les quantités équimoléculaires de l'inosine et du (DL) 5-HTP du commerce en solution aqueuse à une température voisine de 70 °C, en ce qu'on laisse refroidir le mélange réactionnel en 4 à 5 heures à la température ambiante puis en maintenant pendant environ 48 heures à une température voisine de 5 °C et en ce que l'on sépare les cristaux purs du complexe inosine-(L) 5-HTP obtenus.

9. Procédé selon la Revendication 8, caractérisé en ce que les eaux-mères restantes après la filtration des cristaux du complexe inosine-(L) 5-HTP sont portées, pour effectuer la racémisation, à une température comprise entre 150 et 200 °C pendant 3 à 5 heures.

10. Procédé selon la Revendication 9, caractérisé en ce que avant de procéder à la racémisation par chauffage, on isole le 5-HTP présent dans les eaux-mères par passage sur résines échangeuses d'ions du type acide fort, suivi d'une élution par une solution diluée d'hydrates d'hydrazine.

**Claims**

1. Pharmaceutical compositions capable of correcting a deficiency in the metabolism of serotonin,

characterized in that they consist of an association of 5-HTP with purine and/or pyrimidine and/or pyridine bases except for those which are substituted or esterified.

2. A composition according to Claim 1, characterized in that the association 5-HTP/nitrogen heterocycle is an equimolecular association.

3. A composition according to either one of Claims 1 and 2, characterized in that the 5-HTP and the purine and/or pyrimidine and/or pyridine base form a complex.

4. A composition according to either one of Claims 1 and 2, characterized in that the 5-HTP and the purine and/or pyrimidine and/or pyridine base form a salt.

5. A composition according to any one of Claims 1 to 4, characterized in that the 5-HTP can be used in the form of any one of its enantiomers, the (L) form, the (D) form or the (D, L) racemic mixture.

6. A composition according to any one of Claims 1 to 5, characterized in that the base which forms a salt or a complex with the HTP is taken from the group comprising inosine, theophylline, theobromine, allopurinol, hypoxanthine, folic acid, nicotinamide, caffeine and orotic acid.

7. Medicaments consisting of or containing the compositions according to any one of Claims 1 to 6.

8. A process for the preparation of the complex inosine/(L)-5-HTP, characterized in that equimolecular quantities of inosine and commercial (D, L)-5-HTP are reacted in aqueous solution at a temperature of the order of 70 °C, in that the reaction mixture is cooled by leaving it for 4 to 5 hours at room temperature and then by keeping it for about 48 hours at a temperature of the order of 5 °C, and in that the resulting pure crystals of the complex inosine/(L)-5-HTP are separated off.

9. The process according to Claim 8, characterized in that the mother liquors remaining after the crystals of the complex inosine/(L)-5-HTP have been filtered off are heated at a temperature of between 150 and 200 °C for 3 to 5 hours in order to effect racemization.

10. The process according to Claim 9, characterized in that, before effecting the racemization by heating, the 5-HTP present in the mother liquors is isolated by passage over ion exchange resins of the strong acid type, followed by elution with a dilute solution of hydrazine hydrates.

**Patentansprüche**

1. Arzneimittel, die dazu imstande sind, das Stoffwechseldefizit von Serotonin zu korrigieren, dadurch gekennzeichnet, dass sie aus einer Assoziation von 5-HTP mit Purin- und/oder Pyrimidin- und/oder Pyridinbasen bestehen, mit Ausnahme solchen Basen die substituiert oder esterifiziert sind.

2. Arzneimittel nach Anspruch 1, dadurch gekennzeichnet, daß die Assoziation 5-HTP/stickstoffhaltiger Heterocyclus eine äquimolare Assoziation ist.

3. Arzneimittel nach einem der Ansprüche 1 oder 2, dadurch gekennzeichnet, daß das 5-HTP und die Purin- und/oder Pyrimidin- und/oder Pyridinbase einen Komplex bilden.

4. Arzneimittel nach einem der Ansprüche 1 oder 2, dadurch gekennzeichnet, daß das 5-HTP und die Purin- und/oder Pyrimidin- und/oder Pyridinbase ein Salz bilden.

5. Arzneimittel nach einem der Ansprüche 1 bis 4, dadurch gekennzeichnet, daß das 5-HTP in Form eines seiner Enantiomeren, der (L)-Form, der (D)-Form oder des racemischen (D-L)-Gemisches verwendet werden kann.

6. Arzneimittel nach einem der Ansprüche 1 bis 5, dadurch gekennzeichnet, daß der stickstoffhaltige Heterocyclus, der ein Salz oder einen Komplex mit dem HTP bildet, aus der Gruppe Inosin, Theophyllin, Theobromin, Allopurinol, Hypoxanthin, Folsäure, Nicotinamid, Koffein und Orotsäure genommen ist.

7. Medikamente, dadurch gekennzeichnet, daß sie aus den Mitteln nach einem der Ansprüche 1 bis 6 bestehen oder diese enthalten.

8. Verfahren zur Herstellung des Inosin-(L) 5-HTP-Komplexes nach Anspruch 1, dadurch gekennzeichnet, daß man äquimolare Mengen von Inosin und handelsüblichem (DL) 5-HTP in wäßriger Lösung bei einer Temperatur von etwa 70 °C reagieren laßt, das Reaktionsgemisch 4 bis 5 Stunden bei Umgebungstemperatur abkühlen läßt und dann 48 Stunden bei einer Temperatur von etwa 5 °C hält und daß man die erhaltenen reinen Kristalle des Inosin-(L) 5-HTP-Komplexes abtrennt.

9. Verfahren nach Anspruch 8, dadurch gekennzeichnet, daß man die nach Filtration der Kristalle des Inosin-(L) 5-HTP-Komplexes zurückbleibenden Mutterlaugen bei einer Temperatur zwischen 150 und 200 °C 3 bis 5 Stunden lang hält, um die Racemisierung zu bewirken.

10. Verfahren nach Anspruch 9, dadurch gekennzeichnet, daß man vor dem Racemisierungsverfahren durch Erhitzen das in den Mutterlaugen vorhandene 5-HTP durch Durchleiten durch stark saure Ionenaustauscherharze und anschließendes Eluieren durch eine verdünnte Lösung von Hydrazinhydrat isoliert.